# EUROPÄISCHE PATENTSCHRIFT

(11) **EP 2 664 306 B1**
(45) Veröffentlichungstag und Bekanntmachung des Hinweises auf die Patenterteilung: **31.12.2014**
(21) Anmeldenummer: 12168191.0
(22) Anmeldetag: 16.05.2012
(51) Int. Cl.: A61F 5/01, A61F 5/058, A61F 5/30

(54) **Vorrichtung zur Behandlung des Karpaltunnelsyndroms**
Device for treating carpal tunnel syndrome
Dispositif destiné au traitement du syndrome du canal carpien

(43) Veröffentlichungstag der Anmeldung: 20.11.2013
(73) Patentinhaber: GEBA Medical, 78600 Kolbingen (DE)
(72) Erfinder: Hafner, Klaus, 78600 Kolbingen (DE)
(74) Vertreter: Reimann, Silke

(56) Entgegenhaltungen:
- WO-A1-03/017885
- WO-A2-03/007804
- US-A- 6 146 347
- US-A1- 2003 028 136

## Beschreibung

Die Erfindung betrifft eine Vorrichtung und Verfahren zur Behandlung des Karpaltunnelsyndroms der Hand einer Person, die eine Palmarseite mit Thenar und Hypothenar Regionen und eine Dorsalseite gegenüber der Palmarseite aufweist, umfassend eine Handmanschette, die im Querschnitt als ein C-Profil ausgebildet ist, mit einem Thenar Abschnitt zum Kontaktieren und Halten der Thenar Region der Hand, einem Hypothenar Abschnitt, der parallel zum Thenar Abschnitt positioniert ist, zum Kontaktieren und Halten der Hypothenar Region der Hand, einen dazwischen liegenden Dorsal Abschnitt, der mit dem Thenar und Hypothenar Abschnitt verbunden ist; einem Druckpolster, angeordnet entlang dem Dorsal Abschnitt zum Aufbringen von Druck durch eine erste Kraft auf die Dorsalseite der Hand, die gegenläufig einer zweiten und dritten Kraft durch die Thenar und Hypothenar Abschnitte der Handmanschette ist, um eine Vergrößerung des Karpaltunnels zu bewirken.

Das Karpaltunnelsyndrom bezeichnet ein Kompressionssyndrom des nervus medianus im Bereich des Karpaltunnels, der auf der Palmarseite bzw. Handinnenflächenseite des Handgelenks verläuft. Typische Symptome sind auftretende Schmerzen oder Missempfindungen, die von der Hand in den gesamten Arm einstrahlen können. Im fortgeschrittenen Stadium kann es zu einem Muskelschwund im Bereich des Daumenballens, Schwäche beim Zupacken und zu einer Minderung des Tastgefühls kommen. Eine chirurgische Therapie ist verbunden mit den Gefahren und Komplikationen eines operativen Eingriffs.

Es ist bekannt das Karpaltunnelsyndrom auch konservativ zu behandeln. Dabei vermag das Tragen spezieller Nachtschienen oder auch das Anlegen von geformten Stützverbänden die Beschwerden zumindest für eine Zeit lang zu beseitigen oder abzumildern. Eine Alternative dazu sind Schienen, die den Karpaltunnel der Hand öffnen, indem dieser gedehnt und dadurch die querliegenden Bänder verlängert werden. Dieser Prozess verringert den Druck auf die Nerven, was die Schmerzen mildert und die Heilung der Entzündung ermöglicht.

Die Offenlegungsschrift WO 03/017885 A1 beschreibt eine automatische Vorrichtung und Verfahren zur Behandlung des Karpaltunnelsyndroms, das ein Gehäuse zur Aufnahme der Hand des Patienten mit Druckelementen umfasst, die in Kontakt mit dem Hypothenarbereich bzw. kleinfingerseitige Handfläche, Thenarbereich bzw. daumenseitige Handfläche und Dorsalbereich bzw. Handrücken der Hand stehen. Bei dem Gehäuse zur Aufnahme der Hand handelt es sich um ein geschlossenes Gehäuse in O-Form mit einem Loch für den Daumen. Hierdurch ist es ungünstig erforderlich, dass zwei aktive Druckquellen für das erste Druckelement einen Druck auf den Hypothenarbereich und für das zweite Druckelement einen Druck auf den Thenarbereich der Hand aufbringen. Auf der gegenüberliegenden Dorsalseite der Hand sorgt ein Druckpolster für den Gegendruck. Dabei steuert die Steuereinheit die beiden aktiven Druckquellen derart an, dass der Thenar- und Hypothenarbereich der Hand auseinander und um das dorsal angeordnete Druckpolster gezogen wird. Nur in Folge der beiden palmarseitig wirkenden Kräfte und einer dorsalseitig wirkenden Kraft im geschlossenen Gehäuse wird ein Trennen des Handwurzelknochens der Hand bewirkt. Hierbei muss das Gehäuse sehr genau nach der Größe der zu behandelnde Hand bemessen und daraufhin hergestellt sein, sonst verringert sich der Wirkungsgrad.

In der Offenlegungsschrift WO 03/007804 A2 ist eine Vorrichtung und Verfahren zur Behandlung des Karpaltunnelsyndroms mit einem C-förmigen Gehäuse zur Aufnahme der rechten oder linken Hand mit einem Druckelement im dorsalen Abschnitt offenbart. Das Druckelement ist mit einer aktiven Druckquelle verbunden, so dass, wenn die Hand in das Gehäuse eingelegt ist, das Druckelement aktiviert werden kann, um Druck auf der Dorsalseite der Hand auszuüben. Die Vorrichtung enthält starre Abschnitte zur Kontaktierung der Thenar- und Hypothenarbereiche der Hand. Durch die eine abwärts gerichtete Kraft des Druckelements und die Hebelwirkung der starren Abschnitte wird eine Dehnung des Handwurzelknochens bewirkt. Hierbei ist es erforderlich das Gehäuse so zu bemessen und zu formen, dass die Hand des Patienten wirkungsvoll aufgenommen werden kann und durch präzise gesteuerte Querstreckung behandelt werden kann. Wegen seiner Konstruktion aus starrem Material muss das Gehäuse deshalb in den unterschiedlichen Größen S (klein), M (mittel) und L (groß) hergestellt werden. Durch Messung der Handbreite wird die für unterschiedliche Patienten geeignete Größe ermittelt. Die Verwendung der richtigen Größe des Gehäuses ist ein entscheidender Faktor für die Erfolgsaussichten der Behandlung. Ist das Gehäuse zu schmal, wird die zu behandelnde Hand gestaucht und kann durch das Druckelement nicht ausreichend durchgedrückt werden. Hierdurch verringert sich der Wirkungsgrad, weil keine ausreichende Dehnung des Karpaltunnels erfolgt. Trotz genauer Anleitung, wie die Messung der Handbreite zu erfolgen hat, ist diese Methode stark anfällig für Falschmessungen. Das wiederum hat einen vergleichsweise hohen Anteil an Rücksendungen bzw. Austausch der Geräte zur Folge. Wie viele Patienten die Behandlung mit einer für sie geeigneten Manschette durchführen, lässt sich nur schwer überprüfen.

Der Erfindung liegt daher die Aufgabe zugrunde, eine Vorrichtung und Verfahren zur Behandlung des Karpaltunnelsyndroms bereitzustellen, bei der die Größe der Handmanschette durch die zu behandelnde Person an ihre Hand anpassbar ist und deren Handhabung in einer komfortablen und kontrollierten Art und Weise erfolgt.

Diese Aufgabe wird durch eine Vorrichtung gemäß Anspruch 1 und ein Verfahren gemäß Anspruch 15 gelöst.
Ausführungsformen der Erfindung sind in den Unteransprüchen beschrieben.

Die erfindungsgemäße Lösung besteht darin, in der Handmanschette zwei unterschiedlich starke Einlagen anzubringen, die es dem Patienten erlauben, die Größe der Handmanschette anzupassen bzw. zu verkleinern. Der Patient erhält zusammen mit einer einheitlichen, C-förmigen Handmanschette einen Satz von unterschiedlich starken und farblich gestalteten Einlagen. Durch diese Einlagen entfällt für den Patienten das Problem, die für ihn richtige Größe der Handmanschette durch Messung seiner Handbreiten zu ermitteln. Die mitgelieferten unterschiedlich starken Einlagen ermöglichen es dem Patienten, selbständig die für ihn passende Größe durch Ausprobieren anzupassen und bei Bedarf durch Austauschen der Einlagen zu ändern. Vorteilhaft wird so die Handhabung der Manschette für den Patienten vereinfacht und es wird ihm stets die richtige Größe gegeben.

Die unterschiedlich starken Einlagen werden durch Verbindungselemente lösbar an der Handmanschette befestigt. Die Verbindungselemente sind als Schnappverbindungen ausgeführt. Schnappverbindungen nutzen die besonderen Gestaltungs- und Verformungseigenschaften von thermoplastischen Kunststoffen für die Gestaltung einer einfachen, nahezu beliebig lösbaren, wirtschaftlichen Verbindung. Dabei rasten Nocken, Wülste oder Haken der zu verbindenden Teile formschlüssig in entsprechende Hinterschneidungen ein. Die Eindrückkraft hängt vom Fügewinkel und der Reibungszahl ab, die Lösekraft vom Haltewinkel, der bestimmt, ob die Verbindung lösbar ist oder nicht. Die Schnappverbinder der Einlagen können entweder am Außenrand der Handmanschette oder in seitlichen Montagebohrungen der Handmanschette einrasten und sorgen für eine stabile Verbindung.

Die Behandlung des Karpaltunnelsyndroms mit der erfinderischen Vorrichtung erfolgt durch Erzeugung von mechanischem Druck durch das Aufpumpen eines innerhalb der C-förmigen Handmanschette angebrachten Druckpolsters. Durch den erzeugten Druck wird der Handrücken (Dorsalseite), der in der Manschette befindlichen Hand, durchgedrückt und gegen die, der Handinnenfläche (Palmarseite) zugewandte, offene Seiten der Handmanschette gedrückt. Die durch den Druck erzeugte Hebelwirkung dehnt die Handmanschette an der offenen Seite. Vorteilhaft bewirkt der Druck auf die Hand dabei eine Dehnung des auf der Handinnenfläche verlaufenden Karpaltunnels. Verstärkt wird die Dehnung durch die Hebelwirkung der Handmanschette. Die Dehnung des Karpaltunnels mindert den Druck auf die darin verlaufenden Nervenbahnen und lindert die Symptome des Karpaltunnelsyndroms.

Das im Inneren der Handmanschette befestigte Druckpolster wird für die Behandlung durch ein aktives Druckmittel aufgepumpt. In einer ersten Ausführungsform kann die Druckerzeugung manuell mittels eines Sphygmomanometers erfolgen. Dieser besteht aus einem analogen Druckmesser und einer mit der Hand betätigten Handpumpe aus elastischem Material wie beispielsweise einem Gummiball. Das Sphygmomanometer ist über einen Verbindungsschlauch mit dem Schlauchstutzen des Druckpolsters verbunden. Das Druckpolster wird entsprechend der Bedienungsanleitung unter Einhaltung der vorgeschriebenen Behandlungszyklen vom Patienten aufgepumpt. Durch die manuelle Druckerzeugung besteht die Gefahr, dass der Patient in seinem Wunsch, einen möglichst schnellen Behandlungserfolg zu erzielen, nach dem Motto "mehr hilft mehr" verfährt und sowohl den Druck als auch die Dauer der Behandlungszyklen erhöht. Obgleich keine Berichte über konkrete Schädigungen des Patienten vorliegen, besteht die Gefahr einer Schädigung durch Fehlgebrauch der Handmanschette.

Aus diesem Grund ist in einer alternativen Ausführungsform eine elektronische Steuereinheit vorgesehen. Die elektronische Steuereinheit steuert die Behandlung automatisch nach einem vorprogrammierten Ablauf. Diese bildet zusammen mit der Handmanschette eine Einheit, die eine elektrisch betätigte Druckpumpe zur Druckerzeugung umfasst, die direkt mit dem Schlauchstutzen des Druckpolsters verbunden ist.

Nach dem Start der automatischen Behandlung wird das Druckpolster im Inneren der Handmanschette durch die elektrische Druckpumpe auf einen definierten Druck aufgepumpt und die Behandlungszyklen von der elektronischen Steuerung nach einem vorprogrammierten Ablauf gesteuert. Dabei erhält die elektronische Steuerung ein Drucksignal von einem Drucksensor, das dem durch das Druckpolster ausgeübten Druck entspricht. Demzufolge kann die elektronische Druckpumpe bei unter- oder überschreiten des vordefinierte Drucks durch die Steuerung nachgeregelt werden. Zum Starten bzw. Stoppen der automatischen Behandlung ist die elektronische Steuerung mit wenigstens einer Starttaste und einer Stopptaste ausgestattet. Zusätzlich kann die elektronische Steuerung über eine optische bzw. akustische Ausgabevorrichtung Hinweise oder Warnungen zur Kontrolle des vorprogrammierten Ablaufs an den Benutzer ausgeben. Besonders vorteilhaft verhindert die elektronische Steuereinheit durch den automatischen Behandlungsablauf einen möglichen Fehlgebrauch durch den Patienten und vereinfacht die Anwendung der Handmanschette.

Ein entscheidender Faktor für den Erfolg der Behandlung ist dabei die Einhaltung der vorgeschriebenen Behandlungszyklen zur Behandlung des Karpaltunnelsyndroms der Hand. Zuerst wird die Handmanschette durch einen der beiden Handöffnungen an die zu behandelnde Hand angebracht, wobei der Daumen durch die Daumenöffnung gesteckt wird und das Druckpolster Kontakt zum Handrücken (Dorsalseite) hat. Die passenden Einlagen werden nach Bedarf in die Handmanschette eingesetzt.

Danach wird das Druckpolster auf einen ersten Druck aufgepumpt und der Druck eine erste Behandlungsdauer lang gehalten. Anschließend wird der Druck abgelassen und eine Ruhephase abgewartet. Danach wird das Druckpolster erneut auf einen zweiten Druck aufgepumpt und der Druck eine zweite Behandlungsdauer lang gehalten. Anschließend wird der Druck abgelassen.

Vorgesehen ist ein erster Druck von 190 mmHg für eine Behandlungsdauer von 2 Minuten, gefolgt von einer 1-minütigen Ruhephase, gefolgt von einem zweiten Druck von 190 mmHg für eine Behandlungsdauer von 2 Minuten.

Die Erfindung ist nachfolgend anhand von bevorzugten Ausführungsbeispielen unter Bezugnahme auf die Abbildungen beispielhaft erläutert. Dabei zeigt schematisch:
Fig. 1 eine erste Ausführungsform mit elektronischer Steuerung in Explosivdarstellung
Fig. 2 eine alternative Ausführungsform mit Handpumpe und Druckmesser in Explosivdarstellung
Fig. 3 eine Handmanschette mit Druckpolster, Einlagen und elektronischer Steuereinheit im Querschnitt
Fig. 4 eine Handmanschette mit Druckpolster, Einlagen, Handpumpe und Druckmesser in Isometrieansicht
Fig. 5 eine Handmanschette mit elektronischer Steuerung in Draufsicht
Fig. 6 ein vorprogrammierter Ablauf für das elektronische Steuergerät als Flussdiagramm

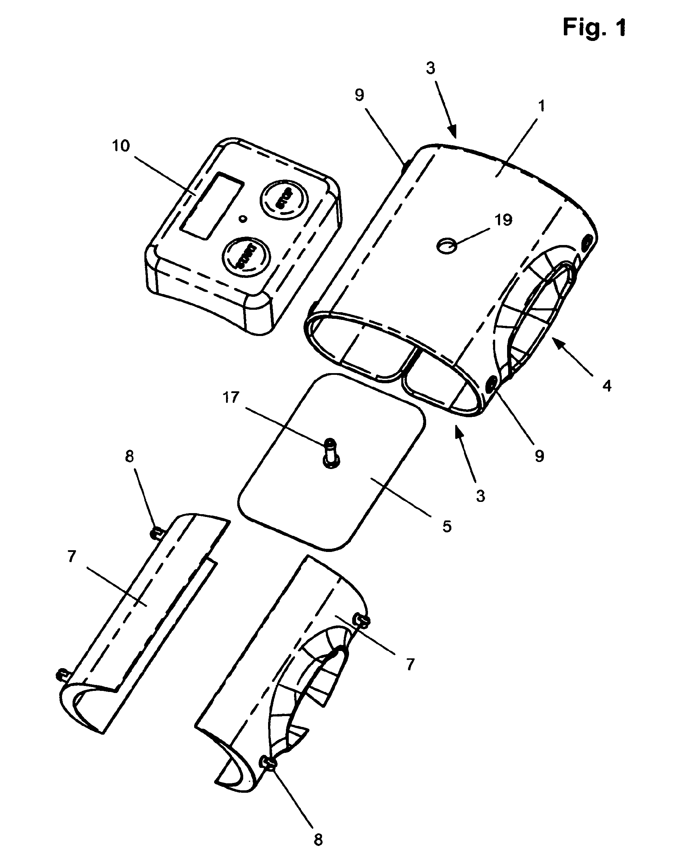

In Fig. 1 ist eine erste bevorzugte Ausführungsform der Handmanschette 1 mit elektronischer Steuerung 10 gezeigt. Die Handmanschette 1 weist ein C-Profil auf, so dass es auf der Palmarseite der Hand getrennt ist. Auf der gegenüberliegenden Seite ist das Druckpolster 5 angeordnet. Die zu behandelnde Hand wird, je nachdem ob es sich um die rechte oder linke Hand handelt, durch eine der beiden Handöffnungen 3 in die Handmanschette 1 eingeführt. Dabei ist darauf zu achten, dass der Daumen durch die seitlich angeordnete Daumenöffnung 4 gesteckt werden kann und die Dorsalseite der Hand mit dem Druckpolster 5 in Kontakt steht.

Auf beiden Seiten weist die Handmanschette 1 jeweils zwei Montagebohrungen 9 auf, in die jeweils die beiden Schnappverbinder 8 der Einlagen 6, 7 formschlüssig einrasten. Das Druckpolster 5 befindet sich innerhalb der Handmanschette 1, wobei der Schlauchstutzen 17 des Druckpolsters 5 durch ein Loch 19 in der Handmanschette 1 nach außen geführt ist. Der Schlauchstutzen 17 führt in die an dieser Seite der Handmanschette 1 anliegende elektronische Steuerung 10 und ist dort mit einer nicht dargestellten elektrischen Druckpumpe verbunden.

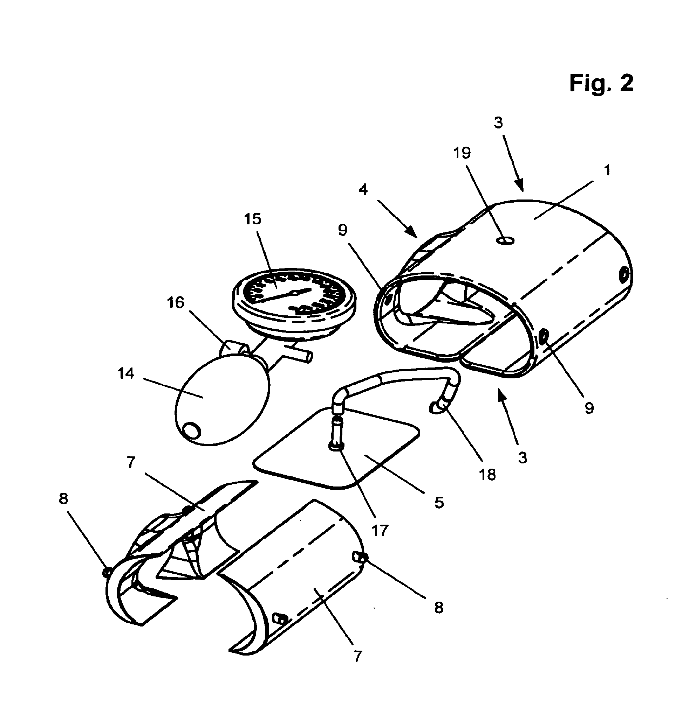

In Fig. 2 ist eine alternative Ausführungsform der in Fig. 1 detailliert beschriebenen Handmanschette 1 mit Sphygmomanometer bestehend aus analogen Druckmesser 15 und Handpumpe 14 gezeigt. Das Sphygmomanometer ist über einen Verbindungsschlauch 18 mit dem Druckpolster 5 verbunden, um das Druckpolster 5 mit Luft zu füllen bzw. über das Ventil 16 Luft hinaus zu lassen. Der Druckmesser 15 misst den Druck im Druckpolster 5 in mm Quecksilbersäule.

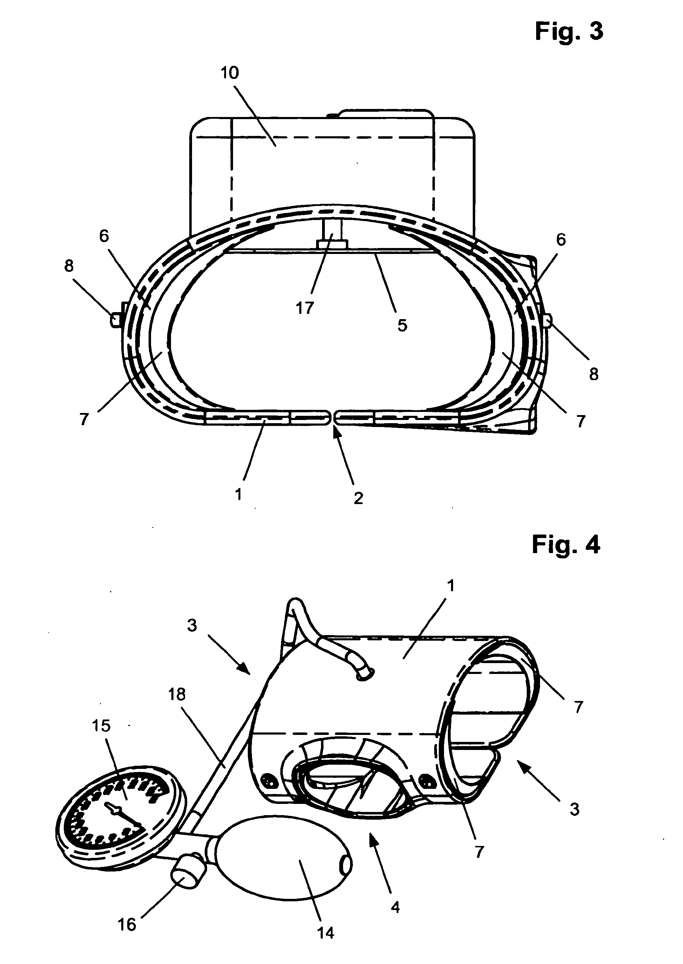

Fig. 3 zeigt die Handmanschette 1 in der ersten Ausführungsform mit elektronischer Steuerung 10 im Querschnitt. Im Inneren der Handmanschette 1 liegt auf der oberen Seite das Druckpolster 5, dessen Schlauchstutzen 17 durch ein Loch in die elektronische Steuerung 10 ragt. Auf der gegenüberliegenden unteren Seite weist die Handmanschette 1 eine Manschettenöffnung 2 auf, so dass die Handmanschette 1 auf der Palmarseite der Hand getrennt ist.

Auf der linken und rechten Seite weist die Handmanschette im Inneren jeweils eine mittlere Einlage 6 und eine große Einlage 7 auf, die je nach Bedarf durch Schnappverbindungen 8, 9 in die Handmanschette 1 eingesetzt werden können. Hierdurch kann die Handmanschette 1 auf die für die Hand des Patienten passende Größe angepasst werden.

Fig. 4 zeigt die in Fig. 2 detailliert beschriebene Handmanschette 1 mit Einlagen 7, Handpumpe 14 und Druckmesser 15 in zusammengesetzter betriebsbereiter Form.

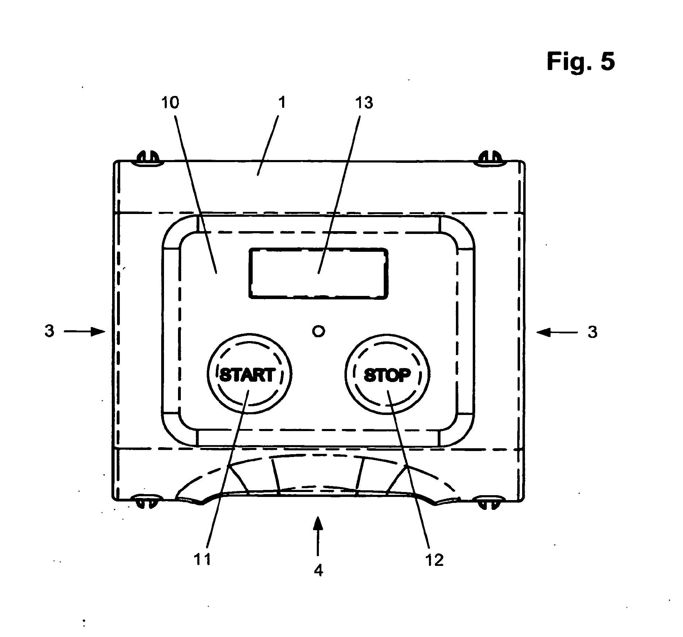

Fig. 5 zeigt die in Fig. 1 detailliert beschriebene Handmanschette 1 mit elektronischer Steuerung 10 in Draufsicht. Auf der oberen Seite der elektronischen Steuerung 10 ist eine optische und/oder akustische Ausgabevorrichtung 13 zur Kontrolle des vorprogrammierten Ablaufs angeordnet. Hierbei kann es sich um ein LCD- bzw. LED-Display, einzelne LEDs oder einen Lautsprecher bzw. Summer handeln. Auf der unteren Seite der elektronischen Steuerung 10 ist eine Eingabevorrichtung bestehend aus einer Starttaste 11 und einer Stopptaste 12 angeordnet.

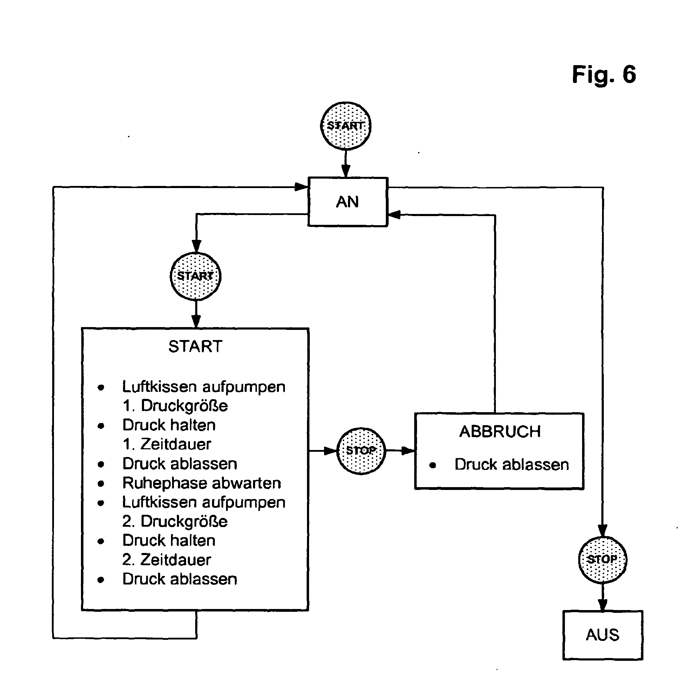

In Fig. 6 ist der vorprogrammierte Ablauf für das elektronische Steuergerät 10 als Flussdiagramm gezeigt. Durch erstmaliges Drücken der Starttaste 11 wird die elektronische Steuerung eingeschaltet und in den Betriebsmodus AN gesetzt. Durch nochmaliges Drücken der Starttaste 11 wird der automatische Behandlungsablauf gestartet und der Betriebsmodus START gesetzt.

Dabei wird das Druckpolster auf einen ersten Druck aufgepumpt und der Druck eine erste Behandlungsdauer lang gehalten. Anschließend wird der Druck abgelassen und eine Ruhephase abgewartet. Danach wird das Druckpolster erneut auf einen zweiten Druck aufgepumpt und der Druck eine zweite Behandlungsdauer lang gehalten. Anschließend wird der Druck abgelassen.

Das Drücken der Stopptaste 12 während des automatischen Behandlungsablaufs führt zu dessen Abbruch, wodurch der Druck abgelassen wird und der Betriebsmodus ABBRUCH gesetzt wird. Das Drücken der Stopptaste 12 außerhalb des automatischen Behandlungsablaufs führt zum Ausschalten der elektronischen Steuerung und der Betriebsmodus AUS wird gesetzt.

### Bezugszeichen

- 1: Handmanschette
- 2: Manschettenöffnung
- 3: Handöffnung
- 4: Daumenöffnung
- 5: Druckpolster
- 6: Mittlere Einlage
- 7: Große Einlage
- 8: Schnappverbinder
- 9: Montagebohrung
- 10: Elektronische Steuerung
- 11: Starttaste
- 12: Stopptaste
- 13: Ausgabevorrichtung
- 14: Handpumpe
- 15: Druckmesser
- 16: Ventil
- 17: Schlauchstutzen
- 18: Verbindungsschlauch

## Patentansprüche

1. Vorrichtung zur Behandlung des Karpaltunnelsyndroms der Hand einer Person, die eine Palmarseite mit Thenar und Hypothenar Regionen und eine Dorsalseite gegenüber der Palmarseite aufweist, umfassend eine Handmanschette (1), die im Querschnitt als ein C-Profil ausgebildet ist, mit einem Thenar Abschnitt zum Kontaktieren und Halten der Thenar Region der Hand, einem Hypothenar Abschnitt, der parallel zum Thenar Abschnitt positioniert werden kann, zum Kontaktieren und Halten der Hypothenar Region der Hand, einen dazwischen liegenden Dorsal Abschnitt, der mit dem Thenar und Hypothenar Abschnitt verbunden werden kann; einem Druckpolster (5), angeordnet entlang dem Dorsal Abschnitt zum Aufbringen von Druck durch eine erste Kraft auf die Dorsalseite der Hand, die gegenläufig einer zweiten und dritten Kraft durch die Thenar und Hypothenar Abschnitte der Handmanschette (1) ist, um eine Vergrößerung des Karpaltunnels zu bewirken,
**dadurch gekennzeichnet,**
**dass** die Vorrichtung mindestens zwei unterschiedlich starke austauschbare Einlagen (6, 7) aufweist, die austauschbar an der Thenar- und der Hypothenarseite der Hand angeordnet sind und die je nach Handform verwendet werden, um den Innendurchmesser der C-förmigen Handmanschette (1) auf unterschiedlich breite Hände der zu behandelnden Person anzupassen.

2. Vorrichtung nach Anspruch 1, **dadurch gekennzeichnet,**
**dass** die unterschiedlich starken Einlagen (6, 7) farblich unterschiedlich gestaltet sind.

3. Vorrichtung nach Anspruch 1, **dadurch gekennzeichnet,**
**dass** die unterschiedlich starken Einlagen (6, 7) durch Verbindungselemente lösbar an der Handmanschette (1) befestigt werden.

4. Vorrichtung nach Anspruch 3, **dadurch gekennzeichnet,**
**dass** die Verbindungselemente als Schnappverbindungen (8) ausgeführt sind, die entweder am Außenrand der Handmanschette (1) oder in seitlichen Montagebohrungen (9) formschlüssig einrasten und für eine stabile Verbindung sorgen.

5. Vorrichtung nach Anspruch 1, **dadurch gekennzeichnet,**
**dass** der Druck auf die Dorsalseite der Hand durch ein aktives Druckmittel erzeugt wird, der das Druckpolster (5) aufpumpt.

6. Vorrichtung nach Anspruch 5, **dadurch gekennzeichnet,**
**dass** das aktive Druckmittel eine manuell betätigte Handpumpe (13) aus elastischem Material ist.

7. Vorrichtung nach Anspruch 5, **dadurch gekennzeichnet,**
**dass** das aktive Druckmittel eine elektrisch betätigte Druckpumpe ist.

8. Vorrichtung nach Anspruch 6, **dadurch gekennzeichnet,**
**dass** die manuell betätigte Handpumpe (13) mit einem Druckmesser (14) zur Überwachung des Drucks verbunden ist.

9. Vorrichtung nach Anspruch 7, **dadurch gekennzeichnet,**
**dass** die elektrisch betätigte Druckpumpe von einer elektronischen Steuerung (10) automatisch gesteuert wird und einen definierten Druck nach einem vorprogrammierten Ablauf aufbaut.

10. Vorrichtung nach Anspruch 9, **dadurch gekennzeichnet,**
**dass** die elektronische Steuerung (10) von einem Drucksensor ein Drucksignal erhält, das dem durch das Druckpolster (5) ausgeübten Druck entspricht.

11. Vorrichtung nach Anspruch 9, **dadurch gekennzeichnet,**
**dass** die elektronische Steuerung (10) eine Eingabevorrichtung mit wenigstens einer Start- und Stopptaste (11, 12) umfasst.

12. Vorrichtung nach Anspruch 9, **dadurch gekennzeichnet,**
**dass** die elektronische Steuerung (10) eine optische und/oder akustische Ausgabevorrichtung (13) zur Kontrolle des vorprogrammierten Ablaufs umfasst.

13. Vorrichtung nach Anspruch 9, **dadurch gekennzeichnet,**
**dass** der vorprogrammierte Ablauf Werte vorbestimmter Druckgröße und vorbestimmter Behandlungsdauer enthält.

14. Vorrichtung nach einem der vorstehenden Ansprüche, **dadurch gekennzeichnet,**
**dass** die Druckpumpe, die elektronische Steuerung (10), die Eingabevorrichtung (11, 12) und die Ausgabevorrichtung (13) in einem gemeinsamen Gehäuse angeordnet sind.

## Claims

1. Device for treating carpal tunnel syndrome of a person's hand, which hand has a palmar aspect with thenar and hypothenar regions and a dorsal aspect opposite the palmar aspect, said device comprising a cuff (1) that is formed in cross section as a C-shaped profile, with a thenar portion, which is in contact with and holds the thenar region of the hand, a hypothenar portion, which can be positioned parallel to the thenar portion and is in contact with and holds the hypothenar region of the hand, a dorsal portion, which lies in between and can be connected to the thenar portion and hypothenar portion, a pressure pad (5) arranged along the dorsal portion for applying pressure to the dorsal aspect of the hand by a first force, which is counter to a second force and third force through the thenar and hypothenar portions of the cuff (1), in order to bring about an enlargement of the carpal tunnel, **characterized in that** the device has at least two exchangeable inserts (6, 7) of different thickness, which are arranged exchangeably on the thenar and hypothenar aspects of the hand and are each used depending on the shape of the hand, in order to adapt the internal diameter of the C-shaped cuff (1) when the hands of the person who is to be treated have different widths.

2. Device according to Claim 1, **characterized in that** the inserts (6, 7) of different thickness are made in different colours.

3. Device according to Claim 1, **characterized in that** the inserts (6, 7) of different thickness are secured releasably on the cuff (1) by connection elements.

4. Device according to Claim 3, **characterized in that** the connection elements are designed as snap-fit connections (8), which latch with a form fit either on the outer edge of the cuff (1) or in lateral assembly bores (9) and ensure a stable connection.

5. Device according to Claim 1, **characterized in that** the pressure on the dorsal aspect of the hand is generated by an active pressure means, which pumps up the pressure pad (5).

6. Device according to Claim 5, **characterized in that** the active pressure means is a manually actuated hand pump (14) made of elastic material.

7. Device according to Claim 5, **characterized in that** the active pressure means is an electrically actuated pressure pump.

8. Device according to Claim 6, **characterized in that** the manually actuated hand pump (14) is connected to a pressure meter (15) for monitoring the pressure.

9. Device according to Claim 7, **characterized in that** the electrically actuated pressure pump is controlled automatically by an electronic control system (10) and builds up a defined pressure according to a pre-programmed sequence.

10. Device according to Claim 9, **characterized in that** the electronic control system (10) receives a pressure signal from a pressure sensor, said pressure signal corresponding to the pressure exerted by the pressure pad (5).

11. Device according to Claim 9, **characterized in that** the electronic control system (10) comprises an input device with at least a start button and stop button (11, 12).

12. Device according to Claim 9, **characterized in that** the electronic control system (10) comprises a visual and/or acoustic output device (13) for monitoring the pre-programmed sequence.

13. Device according to Claim 9, **characterized in that** the pre-programmed sequence contains values of predefined pressure and predefined duration of treatment.

14. Device according to one of the preceding claims, **characterized in that** the pressure pump, the electronic control system (10), the input device (11, 12) and the output device (13) are arranged in a common housing.

## Revendications

1. Dispositif destiné au traitement du syndrome du canal carpien de la main d'une personne qui présente une face palmaire avec des régions thénar et hypothénar et une face dorsale à l'opposé de la face palmaire, comprenant une manchette pour la main (1) qui est réalisée en section transversale sous forme de profilé en C, avec une portion thénar destinée à venir en contact et à retenir la région thénar de la main, une portion hypothénar qui peut être positionnée parallèlement à la portion thénar, destinée à venir en contact et à retenir la région hypothénar de la main, une portion dorsale située entre elles, qui peut être connectée à la portion thénar et à la portion hypothénar ; un coussin de pression (5) disposé le long de la portion dorsale pour appliquer une pression par le biais d'une première force sur la face dorsale de la main, qui est opposée à une deuxième et une troisième force à travers les portions thénar et hypothénar de la manchette pour la main (1), afin de provoquer une augmentation du canal carpien,
**caractérisé en ce que**
le dispositif présente au moins deux inserts interchangeables d'épaisseurs différentes (6, 7) qui sont disposés de manière interchangeables sur la face thénar et hypothénar de la main et qui sont utilisés en fonction de la forme de la main pour adapter le diamètre intérieur de la manchette pour la main en forme de C (1) à différentes largeurs de mains de la personne à traiter.

2. Dispositif selon la revendication 1, **caractérisé en ce que**
les inserts d'épaisseurs différentes (6, 7) sont configurés avec des couleurs différentes.

3. Dispositif selon la revendication 1, **caractérisé en ce que** les inserts d'épaisseurs différentes (6, 7) sont fixés par des éléments de connexion de manière détachable à la manchette pour la main (1).

4. Dispositif selon la revendication 3, **caractérisé en ce que**
les éléments de connexion sont réalisés sous forme de connexions par encliquetage (8) qui s'encliquètent par engagement par correspondance de forme soit au niveau du bord extérieur de la manchette pour la main (1) soit dans des trous de montage latéraux (9) et qui assurent une connexion stable.

5. Dispositif selon la revendication 1, **caractérisé en ce que**
la pression sur la face dorsale de la main est produite par un moyen de pression actif qui gonfle le coussin de pression (5).

6. Dispositif selon la revendication 5, **caractérisé en ce que**
le moyen de pression actif est une pompe manuelle (14) commandée à la main en matériau élastique.

7. Dispositif selon la revendication 5, **caractérisé en ce que**
le moyen de pression actif est une pompe de pression à commande électrique.

8. Dispositif selon la revendication 6, **caractérisé en ce que**
la pompe manuelle (14) commandée à la main est connectée à un manomètre (15) pour contrôler la pression.

9. Dispositif selon la revendication 7, **caractérisé en ce que**
la pompe de pression à commande électrique est commandée automatiquement par une commande électronique (10) et produit une pression définie suivant un schéma préprogrammé.

10. Dispositif selon la revendication 9, **caractérisé en ce que**
la commande électronique (10) acquiert un signal de pression provenant d'un capteur de pression, lequel signal de pression correspond à la pression exercée par le coussin de pression (5).

11. Dispositif selon la revendication 9, **caractérisé en ce que**
la commande électronique (10) comprend un dispositif d'entrée avec au moins une touche marche et une touche arrêt (11, 12).

12. Dispositif selon la revendication 9, **caractérisé en ce que**
la commande électronique (10) comprend un dispositif de sortie optique et/ou acoustique (13) pour contrôler la réalisation du schéma préprogrammé.

13. Dispositif selon la revendication 9, **caractérisé en ce que**
la commande électronique (10) comprend un dispositif d'enregistrement optique et/ou acoustique (13) pour commander le schéma préprogrammé.

14. Dispositif selon l'une quelconque des revendications précédentes, **caractérisé en ce que** la pompe de pression, la commande électronique (10), le dispositif d'entrée (11, 12) et le dispositif de sortie (13) sont disposés dans un boîtier commun.
